# EUROPEAN PATENT APPLICATION

(11) **EP 1 225 221 A1**
(43) Date of publication of application: **24.07.2002**
(21) Application number: 02000280.4
(22) Date of filing: 15.01.2002
(51) Int. Cl.: C12N 13/00, C12N 15/87, C12N 5/04

(54) **Laser-based method for cell wall and membrane perforation**

(30) Priority: 17.01.2001 JP 2001008522; 15.11.2001 JP 2001349532
(71) Applicant: OSAKA UNIVERSITY, Suita City Osaka-Fu (JP)
(72) Inventor: Kobayashi, Akio, Toyonaka City, Osaka (JP); Fukui, Kiichi, Osaka City, Osaka (JP); Harajima, Satoshi, Takatsuki City, Osaka (JP); Fukusaki, Eiichiro, Suita City, Osaka (JP); Kajiyama, Shinichiro, Takatsuki City, Osaka (JP); Okuda, Shinya, Ibaraki City, Osaka (JP); Shoji, Takeshi, Osaka City, Osaka (JP)
(74) Representative: von Hellfeld, Axel, Dr. Dipl.-Phys.

(57) **Abstract**

A method for processing a cell, including the steps of irradiating a cell or a living tissue with a laser beam through an optical fiber, and cutting off, removing or boring a cell wall and/or a cell membrane or an entirety of the cell thus irradiated.

## Description

### Background of the Invention

### (1) Field of the Invention

The present invention relates to a method for processing cells and living tissues. Particularly, the invention relates to a method for processing a living sample, wherein a cell and/or a living tissue is processed through being irradiated with a laser beam through an optical fiber.

### (2) Description of Related Art

As conventional methods for processing living bodies by using laser beams, for example, there is known a laser micro-dissection method in which a sample such as a chromosome is irradiated with a laser beam through a medical laser knife or an objective lens of a microscope and thereby a portion of the sample irradiated is cut off. Further, as a method for introducing a foreign matter such as a gene into a cell with use of a laser, there is known a method as disclosed in JP-B 62-7837.

With progress in the biotechnology, a demand has recently been increasing for a method of finely processing cells, wherein a specific cell of a specific tissue is taken as a target. For example, it is investigated that a fine hole is bored in a cell of a meristematic tissue such as shoot apex tissue or a root apical meristem, and a transformed body is directly prepared, without dedifferentiation or dedifferentiation in case of a plant, by directly introducing a foreign matter into that cell through the fine hole. Molecular breeding of many useful plants of which dedifferentiation or dedifferentiation system has not been established is being expected. If cells can be processed in this manner, not only genes but also foreign matters other than the genes, such as organelle including chloroplasts, nuclei, chromosomes and mitochondrions, physiologically active substances, indicating agents, or functional proteins will be able to be introduced into cells. Then, diagnosis and curing of diseases, molecular breeding of agricultural products imparted with various tolerances, production of useful living matters including livestock, etc. will be possible.

From this point of view, among conventional methods for processing living bodies with laser, the laser knife method has a problem in that it cannot finely process one cell or so by removing a cell wall and/or a cell membrane, since it gives a large output, but largely hurts cells due to large thermal damages. On the other hand, although the laser micro-dissection method can process one cell or so, it has problems in that since laser is irradiated through the objective lens under the microscope, a preliminary treatment of a sample, such as preparation of a cut piece, etc. is required for the laser irradiation and that a plant body itself having a complicated three-dimensional shape, such as a meristematic tissue of a shoot apex, cannot be processed through irradiating with laser for the introduction of a foreign matter thereinto. As foreign matter-introducing methods not relying upon laser, a micro-injection method, a liposome fusion method, an electropolation method, etc. may be recited. When employed for plant cells, the electropolation method and liposome fusion method require protoplasto preparation, so that they have a problem that it is difficult to introduce a foreign matter into one specific cell of a specific tissue. The micro-injection method has a problem in that it requires skill in treating a plant cell having a cell wall tougher than that of an animal cell, and thus such treatment is extremely difficult.

Therefore, a demand has existed for developing a method which is easy in operation, is not limited to specific kinds of living things, can be generally and widely employed, and enables fine processing for the introduction of a foreign matter into a specific site or cell of a living tissue having a complicated three-dimensional shape. However, such a cell-processing method has not been known up to now.

### Summary of the Invention

Under these circumstances, it is an object of the present invention to provide a method for processing a cell by utilizing high processability of a laser beam and characteristics of an optical fiber.

The present inventors made investigations to solve the above problems, and consequently discovered that an appropriate site of a cell, particularly a tissue having a complicated three-dimensional shape, can be processed by utilizing high processability of the laser beam and characteristics of the optical fiber. The inventors thus accomplished the present invention based on this discovery.

That is, the method for processing a cell according to the present invention comprises the steps of irradiating a cell or a living tissue with a laser beam through an optical fiber, and cutting off, removing or boring a cell wall and/or a cell membrane or an entirety of the cell thus irradiated.

The following are preferred embodiments of the present invention, and any combinations thereof are also preferred embodiments of the invention, unless contradictory to each other or one another.
In a preferred embodiment of the cell-processing method in the present invention, the laser beam has a wavelength of 500 nm or less.

In another preferred embodiment of the cell-processing method in the present invention, the cell is irradiated with the laser through reflection and condensing.

In other preferred embodiment of the cell-processing method in the present invention, the reflection and condensing are effected through a chip of quartz glass. The quarts glass chip may be hollow or solid. In case of the solid glass chip, the reflection and condensing are effected at the outer interface between the exterior. In case the hollow glass chip, the reflection and condensation are effected at the inner wall and/or inner wall surfaces thereof.
In a further preferred embodiment of the cell-processing method in the present invention, a surface of the quartz glass chip is coated with a metal.

In a still further preferred embodiment of the cell-processing method in the present invention, the coating metal is at least one metal selected from the group consisting of aluminum, platinum, gold, palladium and/or oxides thereof.

In a still further preferred embodiment of the cell-processing method in the present invention, the laser is at least one laser selected from the group consisting of an YAG laser, an excimer laser, an Ar ion laser, a nitrogen laser and a nitrogen-exited laser.

In a further preferred embodiment of the cell-processing method in the present invention, after a part of the cell membrane and/or cell wall of the cell is bored by irradiation with the laser beam, a foreign matter is introduced into the cell through a laser-irradiated portion thereof.

In a still further preferred embodiment of the cell-processing method in the present invention, the foreign matter is at least one selected from the group consisting of a genetic substance, a protein, an organelle, a physiologically active substance and an indicating agent.

In a still further preferred embodiment of the cell-processing method in the present invention, the genetic substance is at least one selected from the group consisting of a DNA, an RNA, an oligonucleotide, a plastid, a chromosome, an artificial chromosome, an organelle DNA, and a nucleic acid analogue.
In a still further preferred embodiment of the cell-processing method in the present invention, the optical fiber is hollow.
In a still further preferred embodiment of the cell-processing method in the present invention, a hollow space of the optical fiber is filled with an inert gas.
In a still further preferred embodiment of the cell-processing method in the present invention, the inert gas is at least one gas selected from the group consisting of a nitrogen gas, an argon gas and a helium gas.
In a still further preferred embodiment of the cell-processing method in the present invention, a wall surface of a hollow space of the optical fiber is coated with a metal.

A second aspect of the present invention is to provide a transformed body, wherein a genetic substance is introduced into a cell by using the method in claim 9.
These and other objects, features and advantages of the invention will be appreciated upon reading of the following description of the invention when taken in conjunction with the attached drawings, with the understanding that any modifications, variations and changes of the invention will be easily made by a skilled person in the art to which the invention pertains.

### Brief Description of the Drawings

For a better understanding of the invention, reference is made to the attached drawings, wherein:
Fig. 1 is a schematic view of a quartz capillary vapor deposited with aluminum;
Fig. 2 is a schematic view of a flexible optical fiber type irradiating system;
Fig. 3 is a view showing a shape of an epidermis of an onion processed by irradiating it with laser;
Fig. 4 is a view showing the relationship between a fiber-bending angles and specific points.
Fig. 5a gives a bright field-observed image of an epidermis of the onion before processing with laser;
Fig. 5b gives a bright field-observed image of an epidermis of the onion during processing with laser; and

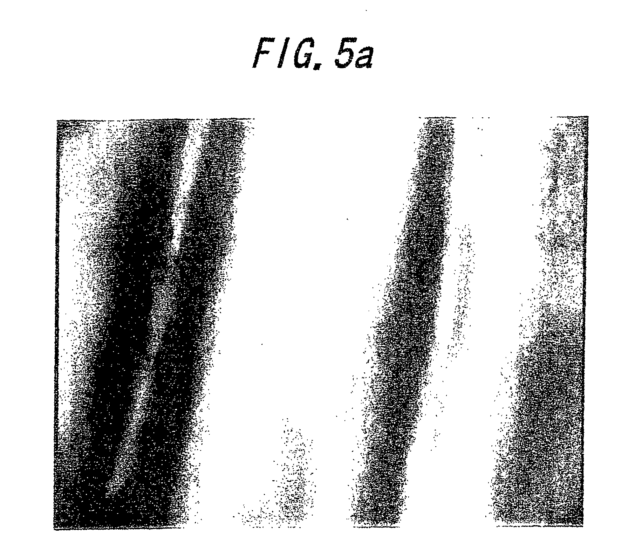

Fig. 5a gives a bright field-observed image of an epidermis of the onion after processing with laser.

### Detailed Description of the Invention

According to the cell-processing method of the present invention, the cell wall and/or the cell membrane of the cell is cut off, removed or bored by irradiating it with a laser beam through an optical fiber. As the cells to which the present invention is applicable, animal cells, plant cells, microorganisms, etc. may be recited, but the invention is not particularly limited to any cells.

In the present invention, the optical fiber is used. The reason why the optical fiber is used in the present invention is that the irradiating direction, the condensability, the irradiating angle, etc. of the laser beam can be easily controlled by the optical fiber. The optical fiber means a dielectric linear path intended to transmit a visual light or a near infrared light therethrough. The optical fiber, which is not particularly limited, is a wide concept encompassing a synthetic quartz fiber, a hollow fiber, etc. as mentioned later.

As the optical fiber to be applicable in the present invention, an optical fiber having a high transmittance for an ultraviolet laser is preferred, although not particularly limited thereto. Since the transmittance of the optical fiber differs depending upon the wavelengths of the lasers, an optical fiber having a high transmittance for a laser beam having a specific wavelength is preferred. The reason why the optical fiber having a high transmittance is preferred is that such an optical fiber does not decrease the energy of the laser.

A core material for the optical fiber requires that (1) the material has such a transmittance as to effectively transmit the laser beam, and (2) it has such a strength as not being destroyed with the energy of the laser beam introduced.

The transmittance of the optical fiber differs depending upon the wavelength of the laser used. In case that the wavelength of the laser is not more than 500 nm, the transmittance is preferably 10 to 60% per meter. More preferably, it is 30 to 60 % per meter. The transmittance of the optical fiber can be increased by increasing an amount of hydroxide groups in quartz through introducing the groups into the quartz. The transmittance can be set at a desired level by adjusting the content of the hydroxide groups.

The configuration of the optical fiber may be hollow. The shape of the hollow space is not particularly limited, so long as an inert gas can be flown therethrough. As the hollow optical fiber, an optical fiber made of fused silica may be recited, for example.

In a preferred embodiment of the present invention, the hollow space of the optical fiber is filled with the inert gas. This is because when the interior of the optical fiber is filled with (replaced by) the inert gas, the density of the energy emitted from the optical fiber can be made greater than that in the case where the interior of the optical fiber is under atmosphere.

No limitation is posed upon such an inert gas, but an argon gas, a nitrogen gas, a helium gas, etc. may be recited, for example. At least one kind of these gases can be used. The nitrogen gas is preferred as the inert gas from the standpoint that the gas is inexpensive and readily available, and can enhance the transmittance of the laser.

Further, the wall surface of the interior space may be coated with a metal. No limitation is posed upon the coating metal, but at least one kind selected from the group consisting of aluminum, platinum, gold, palladium and/or oxides thereof may be recited. The metal is preferably aluminum from the standpoint of view that it has excellent reflection efficiency for the excimer laser. When the interior of the optical fiber is coated with aluminum, the excimer laser beam of 193 nm can be effectively transmitted. The wavelength of 193 nm affords a more excellently processed state of a plant cell wall as compared with other wavelengths. Therefore, the laser having the wavelength of 193 nm is preferably used. Further, if the wavelength is too short, the laser tends to be a vacuum ultraviolet laser, which is difficult to handle. On the other hand, if the wavelength exceeds 200 nm, a portion which is irradiated with laser is thermally processed, so that the processed portion tends to be not so clean.

If the wavelength of the laser exceeds 500 nm, a preferable transmittance is 70 to 90 % per meter.
No limitation is posed upon the laser to be used in the present invention. The laser is excellent in that its light condensability is extremely exceeding and almost no thermal effect exists in a portion other than the laser beam spot. As the laser, the YAG laser, the excimer laser, the Ar laser, the nitrogen laser, the nitrogen-excited laser, etc. may be recited. The excimer laser is preferred from the standpoint of view that it causes a particularly small thermal damage upon a processed cell, has an excellent processing precision, and can easily control a processed depth by the irradiation energy and the number of irradiations.

The irradiation condition of the laser upon the cells can be appropriately varied depending upon the kinds of the cells.
The laser spot diameter is in a range of 0.5 to 100 µm, for example. Preferably, it is in a range of 2 to 10 µm. These ranges are selected as being large enough to introduce a foreign matter after the irradiation and cause a smaller damage upon the cell.

No particular limitation is posed upon the density of the energy of the laser, either. However, it may be in a range of 1 to 100 mJ/cm², and preferably in a range of 30 to 80 mJ/cm². The reason for the above range is that if it is less than 1 mJ/cm², the cell wall cannot be sufficiently processed, whereas if it is more than 100 mJ/cm², the laser penetrates the cell membrane and largely damages the cell.

Particularly, in order to completely remove the cell wall by laser irradiation at one time, irradiation can be effected in a higher range of the energy density than as recited above. A preferable range in this case is 500 to 700 mJ/cm². The reason why the energy density is set in such a higher range in case that the cell wall is removed by the irradiation at one time is that if it is less than 500 mJ/cm², the cell wall cannot be certainly penetrated, whereas if it is more than 700 mJ/cm², the living percentage of the cell after the irradiation decreases, although the cell wall can be penetrated.

The output of the laser is preferably 1 to 1000 mJ/cm², more preferably 10 to 100 mJ/cm².

No limitation is posed upon the size of the hole bored by the irradiation with the laser, which size depends upon that of a foreign matter to be introduced. For example, the size of the hole is about 1 to 1000 µm². In case that a relatively large substance such as an organelle is to be introduced, the size of hole is around 100 to 1000 µm². In case that a relatively small substance such as a genetic substance is to be introduced, the size of hole is around 1 to 100 µm².

Considering in connection with the wavelength of the laser beam that the processing precision is enhanced and the damage upon the cell is as small as possible, the laser having a shorter wavelength is preferably used. For example, the wavelength is preferably not more than 500 nm.

Under the above conditions, the laser beam is irradiated to bore a part of the cell membrane and/or the cell wall of the cell, so that a foreign matter can be introduced into the cell through the hole thus bored. Further, the cell can be converted to a protoplast or a spheroplast by cutting off or removing a part of or an entire part of the cell membrane and/or the cell wall of the cell. In the present invention, since the laser is irradiated upon the cell through the optical fiber, only a specific cell existing in a complicated tissue can be converted to a protoplast or a spheroplast, such a cell only can be transformed.

The laser beam may be introduced directly into the optical fiber or introduced into the optical fiber after being once condensed with a lens so as to enhance the condensability of the laser beam.

Further, in the present invention, the laser beam may be irradiated through reflection and condensing. In case that the laser beam is irradiated through the optical fiber, the density of energy is decreased. For this reason, the density of the energy can be increased through reflection and condensing. The density of energy can be converged by attaching a glass tip or quartz chip to a tip of the optical fiber. The density of energy can be more effectively converged by coating the surface of the glass chip or the quartz chip with a metal such as aluminum, gold, platinum or palladium and/or oxides thereof.

As mentioned above, according to the foreign matter-introducing method of the present invention, an organelle such as a nucleus or a chromosome from the same or different living thing or a huge DNA such as an artificially prepared chromosome can be introduced into the cell. Therefore, a one-time introduction of multiple chromosomes artificially prepared, which has been difficult up to now, can be effected. For example, a group of genes participating in the biosynthesis of a useful physiologically active substance can be introduced at one time, so that the useful physiologically active substance can be effectively produced in a living thing growing fast.

As also mentioned above, according to the foreign matter-introducing method of the present invention, a plant hormone can be introduced into a plant. Therefore, if growth of a tissue near a portion into which the foreign matter is introduced is to be controlled, an auxin such as indoleacetic acid or naphthaleneacetic acid, cytokinin such as zeatin or kinetin, abscisic acid, gibberellin or peptide-based hormone is introduced into a plant cell by the foreign matter-introducing method of the present invention, thereby controlling the growth of the specific site of the plant.

When an antibacterial material such as phytoalexin more specifically, pisatin, medicarpin, rishitin, or rishitinol is introduced into a cell in the same manner as mentioned above, a disease tolerance of a tissue liable to be infected with a bacterial can be enhanced. Further, when an active oxygen-removing agent such as phytochelatin or glutathione is added, tolerance against UV and light in a light-receiving tissue such as a leaf and tolerance against stress such as heavy metals in roots can be enhanced.

### (Examples)

In the following, the present invention will be concretely explained in more detail based on examples, but the invention is not intended to be interpreted as being limited to these examples.

### Example 1

### (1) Preparation of quartz capillary

Capillary was produced from a quartz glass tube Q100-70-10 manufactured by SUTTER INSTRUMENT COMPANY (S.I.C.) with use of P-200 Laser powered quartz micropipette puller of S.I.C. Inc.

### (2) Vapor deposition of aluminum on quartz capillary

Aluminum was vapor deposited on the surface of the quartz capillary produced in above (1) by a vacuum vapor deposition apparatus (manufactured by Shinkuu Kikou Co., Ltd.) with use of aluminum (manufactured by The Nilaco Corporation) as a vapor deposition source. The thickness of the vapor deposited aluminum was set at about 200 nm. In order to completely vapor depositing the quartz capillary, vapor deposition was effected totally three times each by 120°.

### (3) Introduction of 193 nm laser beam into special quartz fiber

A 193 nm laser beam was introduced into a special quartz fiber prepared by doping an ordinary quartz fiber with hydroxyl groups. Measurement of the density of the energy of the laser at an outlet of the fiber having a diameter of 200 µm gave 2 µJ.

### (4) Condensing of laser beam by the aluminum-vapor deposited quartz capillary prepared in above (2)

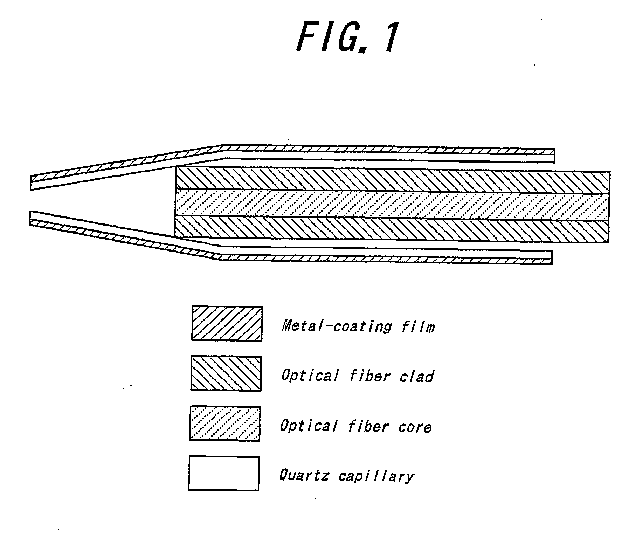

The aluminum-vapor deposited quartz capillary prepared in above (2) was attached to the outlet of the special quartz fiber prepared in above (3). The energy of the laser emitted from a tip of the capillary was 25 nJ. Fig. 1 shows a concept view of the aluminum-vapor deposited quartz capillary.

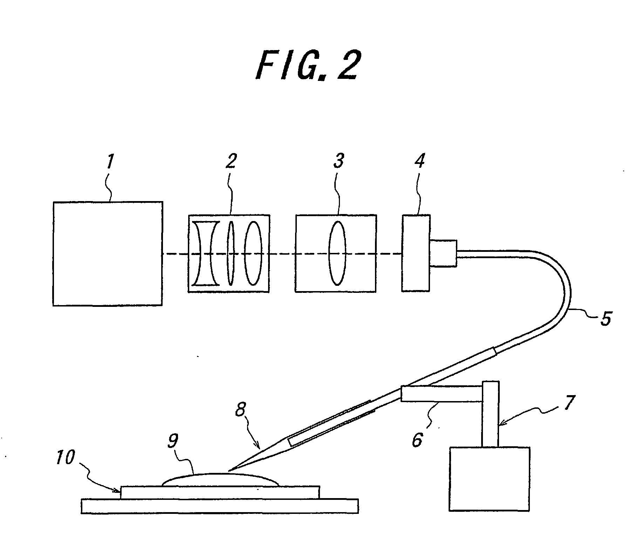

The special quartz fiber to which the aluminum-vapor deposited quartz capillary was attached was fitted to a three-dimensional manipulator manufactured by Eppendorf Inc., and a laser-irradiating portion was made freely movable. Fig. 2 is a schematic view of a flexible fiber-irradiating system. In Fig. 2, 1 is an ArF excimer laser, 2 shape-rectifying optical system, 3 a laser-introducing lens, 4 an optical fiber adaptor, 5 an optical fiber, 6 a supporting rod, 7 a three-dimensional manipulator, 8 an aluminum-vapor deposited capillary, 9 an onion epidermic cell, and 10 an MS agar culture medium.

### (5) Processing of onion epidermic cell through irradiation with laser

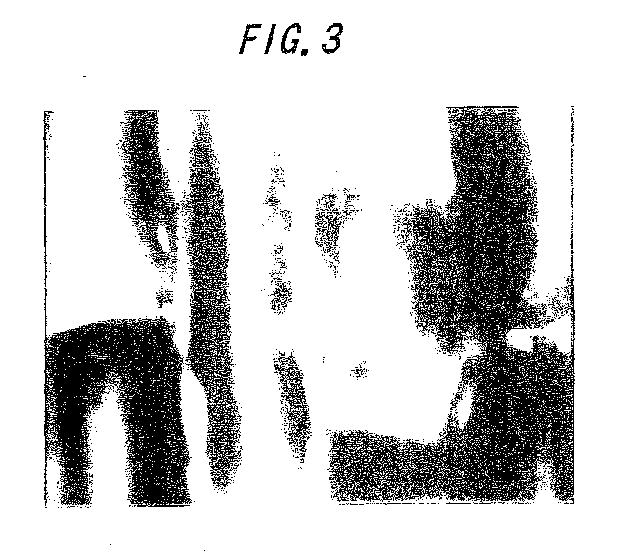

An epidermic cell was peeled off from an onion hydroponically cultivated for 2 days, and placed on an MS agar culture medium. A target cell to be process was selected under a microscope, and a laser-irradiating portion attached to the three-dimensional micromanupilator manufactured by Eppendorf Inc. was contacted with the target cell to irradiate laser to the cell at 10 Hz for 60 seconds. Consequently, a cell wall of the target cell was removed. Observation of the processed shape of the cell wall after the irradiation with the microscope revealed that the cell wall was effectively removed and no damage was given upon the other tissues. Fig. 3 is a figure showing the shape of the onion epidermis processed by irradiation with laser. Further, after the irradiated cell was cultured for 24 hours, its life and death was judged with MTT, which confirmed that the irradiated cell was still alive.

### Example 2

Next, a test was effected by using a hollow fiber manufactured by Fused Silica Inc. as a hollow fiber.
In order to enable flexible handling of an excimer laser, the hollow fiber manufactured by Fused Silica Inc. was used. An IC substrate processing machine L5912 manufactured by Hamamatsu Photonics Inc. was used as a base machine for a laser oscillator. A mirror was placed on a laser optical path of L5912, and the laser path was bent at 90°. The laser beam condensed by passing it through a condensing lens was introduced into the hollow fiber manufactured by Fused Silica Inc. The interior of the hollow fiber of Fused Silica Inc. used was coated with aluminum, so that the excimer layer beam was effectively and successfully reflected. Further, since the interior of the hollow fiber of Fused Silica was replaced by nitrogen gas, the intensity of the energy of the laser emitted from the fused silica hollow fiber was enhanced by 25%.

In order to examine the relationship between the intensity of the laser energy and the bending angle of the optical fiber, how much the laser energy increased when the fiber was filled with the inert gas was examined.

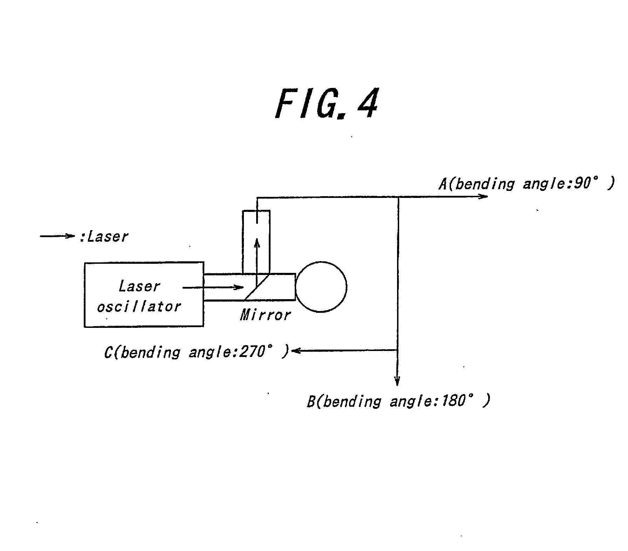

The intensity of an excimer laser emitted from a tip of a hollow optical fiber after the excimer laser was introduced into the optical fiber was measured by a power meter with respect to the bending angle of the optical fiber. Fig. 4 schematically shows bending angles of the optical fiber and measuring points. Since the energy of the excimer laser is decreased through the consumption of the laser energy with ozone generated during the passage of the laser in air, how much the energy was decreased by the replacement of N₂ gas was also examined. While the height was tried to be not changed, the laser energy was measured once per 10 seconds with the power meter, and measured values were recorded. Results are shown in Table 1.

**Table 1**

| | No N₂ gas | N₂ gas filled | Increased energy (%) |
|---|---|---|---|
| A (bending angle 90°) | 3.92 | 6.97 | 17.7 |
| B (bending angle 180°) | 2.47 | 5.23 | 21.1 |
| C (bending angle 270°) | 1.67 | 3.25 | 19.4 |
| Unit: µJ | | | |

A quartz glass chip was attached to the hollow optical fiber manufactured by Fused Silica Inc. at about 270°so that the laser beam emitted from the optical fiber might be condensed. The quartz glass chip used at that time was prepared by a horizontal type capillary-forming device (SUTTER INSTRUMENT Co. Model P-2000) such that its tip diameter was worked at about 1 µm. By using a vacuum vapor depositing apparatus, aluminum was vapor deposited on the outer surface of the quartz glass chip prepared. The vapor deposition was effected in the state that the quartz glass was rotated inside the vacuum vapor deposition apparatus. The surface of the quartz glass chip was uniformly covered with aluminum through vapor deposition under rotation. The thickness (film thickness) of the vapor deposited aluminum was about 200 to 300 nm. The aluminum film vapor deposited needs to have a sufficient thickness, because if the film thickness is small, the reflection efficiency of the excimer laser beam decreases to reduce the intensity of the laser energy emitted from the tip of the quartz glass chip. The laser-irradiating portion of this optical fiber system was contacted with an epidermic cell of an onion by operating the three-dimensionally driven manipulator (Eppendorf 5171). When laser was irradiated in this state, a laser-processed fine hole having a diameter of 2 µm was observed at the onion epidermic cell.

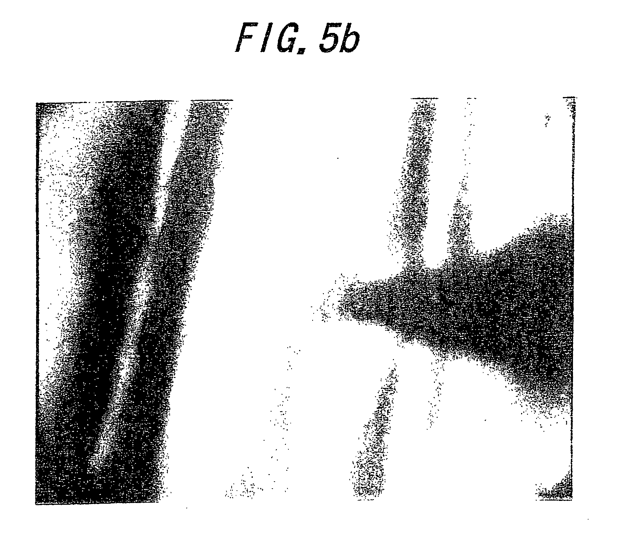

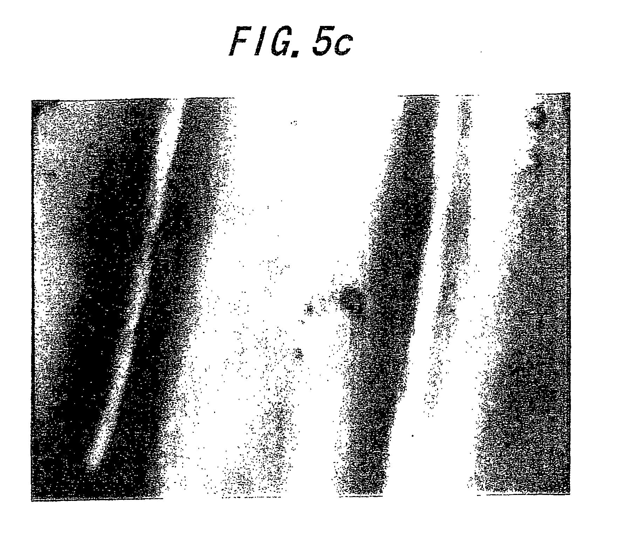

Fig. 5 shows a processed state through the optical fiber. Fig. 5a gives a bright field-observed image of the onion epidermic cell before processing with laser. Fig. 5b gives a bright field-observed image of the onion epidermic cell during processing with the laser by using the hollow optical type laser-irradiating portion. Fig. 5a gives a bright field-observed image of the onion epidermic cell after processing with laser.
A solution of a plasmid DNA was tried to be introduced into the cell through the fine hole thus processed, according to the microinjection method. After the introduction, the onion epidermic cell was cultivated for 24 hours, and a transient expression of the introduced gene was observed by using a fluorescent microscope.

As an genetic information material used as a foreign matter, a chimera gene in which a green fluorescence protein (GFP) encoding a green fluorescence protein of the Gelly Fish (*Aequorea Victoria*) was connected to a downstream side of a 35S promoter of a cauliflower mosaic virus was specifically employed. When this gene is introduced in a plant cell and genetically expressed, the protein generating the green fluorescence is produced. Whether the gene was expressed or not was judged through observation of the light emission with use of the plasmid DNA having this chimera gene. The fluorescence was observed by using an erected image-type fluorescent microscope (BX-50) manufactured by Olympus Co., Ltd. Bright-field images and fluorescent images were observed by using this microscope. A fluorescent filter used in the fluorescent observation was a filter (U-MWIB2, U-MWIBA2, U-MNIBA2).

The cell-processing method according to the present invention has an advantage that a specific cell or a group of specific cells can be used as a target in the production of a transgenic plant through the introduction of the material or the gene.

The cell-processing method according to the present invention also has an advantage that since the target plant species and/or the target tissues to be processed by the irradiation with the laser are not limited, the method has a wide range of applicability.

The cell-processing method according to the present invention has a further advantage that since the irradiating angle can be controlled by the optical fiber, the living cell wall and/or cell membrane can be cut off, removed or bored at an appropriate position.

## Claims

1. A method for processing a cell, comprising the steps of irradiating a cell or a living tissue with a laser beam through an optical fiber, and cutting off, removing or boring a cell wall and/or a cell membrane or an entirety of the cell thus irradiated.

2. The method set forth in claim 1, wherein the laser beam has a wavelength of 500 nm or less.

3. The method set forth in claim 1 or 2, wherein the cell is irradiated with the laser through reflection and condensing.

4. The method set forth in claim 3, wherein the reflection and condensing are effected through a chip of quartz glass.

5. The method set forth in claim 4, wherein a surface of the quartz glass chip is coated with a metal.

6. The method set forth in claim 5, wherein the coating metal is at least one metal selected from the group consisting of aluminum, platinum, gold, palladium and/or oxides thereof.

7. The method set forth in any one of claims 1 to 6, wherein the laser is at least one laser selected from the group consisting of an YAG laser, an excimer laser, an Ar ion laser, a nitrogen laser and a nitrogen-exited laser.

8. The method set forth in any one of claims 1 to 7, which further comprises a step of introducing a foreign matter into the cell and/or the living cell through a laser-irradiated portion thereof after irradiation with the laser beam.

9. The method set forth in claim 8, wherein the foreign matter is at least one selected from the group consisting of a genetic substance, a protein, an organelle, a physiologically active substance and an indicating agent.

10. The method set forth in claim 9, wherein the genetic substance is at least one selected from the group consisting of a DNA, an RNA, an oligonucleotide, a plasmid, a chromosome, an artificial chromosome, an organelle DNA, and a nucleic acid analogue.

11. The method set forth in any one of clams 1 to 10, wherein the optical fiber is hollow.

12. The method set forth in claim 11, wherein a hollow space of the optical fiber is filled with an inert gas.

13. The method set forth in claim 12, wherein the inert gas is at least one gas selected from the group consisting of a nitrogen gas, an argon gas and a helium gas.

14. The method set forth in any one of claims 11 to 13, wherein a wall surface of a hollow space of the optical fiber is coated with a metal.

15. A transformed body, wherein a genetic substance is introduced into a cell by using the method in claim 10.
